# EUROPÄISCHE PATENTANMELDUNG

(11) **EP 2 960 323 A1**
(43) Veröffentlichungstag der Anmeldung: **30.12.2015**
(21) Anmeldenummer: 14174066.2
(22) Anmeldetag: 26.06.2014
(51) Int. Cl.: C12M 1/107, C12M 1/06, C12M 1/42, C02F 1/36

(54) **Vorrichtung und Verfahren für den Aufschluss von biologischen Substraten**

(71) Anmelder: BANDELIN patent GmbH & Co. KG, 12207 Berlin (DE)
(72) Erfinder: Bandelin, Jochen, 14129 Berlin (DE)
(74) Vertreter: Pfenning, Meinig & Partner GbR

(57) **Zusammenfassung**

Die Erfindung betrifft eine Vorrichtung für den Aufschluss von biologischen Substraten unmittelbar in offenen oder geschlossenen Fermentern und/oder in Behältern zur Separation von Feststoffen. Dabei sind eine oder mehrere Ultraschalleinheiten in dem Fermenter und/oder dem Behälter zur Separation von Feststoffen angebracht, und der Fermenter und/oder der Behälter zur Separation von Feststoffen weisen für sich genommen einen Rauminhalt von mindestens 1 m³ auf.

Außerdem betrifft die Erfindung ein entsprechendes Verfahren. Mit der Erfindung wird erreicht, dass der Aufschluss von biologischen Substraten auch großindustriell kostengünstig und störungsfrei durchgeführt werden kann.

## Beschreibung

Die Erfindung betrifft die mechanische Zerkleinerung von biologischem Substrat mittels Ultraschall innerhalb eines Fermenters. Hierbei wird das biologische Material, wie etwa Maissilage, Gülle oder Klärschlamm, durch die mechanischen Scherkräfte des Ultraschalls für den Abbau durch Mikroorganismen aufgeschlossen. Ziel des Einsatzes der Ultraschalltechnologie ist die energieeffiziente Steigerung des Biogasertrages und die Reduzierung der Gärrückstände in landwirtschaftlichen Biogasanlagen und Klärwerken. Insbesondere bei Klärwerken kann das Verfahren sowohl in der aeroben, als auch in der aneroben Vergärung zur Beschleunigung der Abbauprozesse eingesetzt werden.

Der Einsatz von Ultraschallsystemen zur Steigerung des Gasertrags und Reduzierung der Gärrestmenge bei landwirtschaftlichen Biogasanlagen und kommunalen Klärwerken ist in der DE 10 2005 030 895 A1 erwähnt. Hierbei wird ein Teilstrom des Fermenterinhalts einer Biogasanlage durch einen rohrförmigen Reaktor geleitet und durch von außen angelegte Ultraschallschwingsysteme beschallt.

Eine weitere Methode zur Beschallung von biologischen Schlämmen wird in DE 195 17 381 C1 beschrieben. Hierbei wird ein Teilstrom des Fermenters durch ein separates Gefäß geleitet und mittels mehrerer stabförmiger Schwingsysteme, welche in die Suspension eintauchen, mit Ultraschall behandelt.

In der Praxisanwendung hat sich gezeigt, dass eine Beschallung des Teilstromes eines Fermenters mit einem Energieeintrag von 3-5 kWh/m³, bezogen auf die Stromaufnahme des Ultraschallgenerators, und einem Volumenstrom von etwa 1000 I/h bei Fermentern von 2.200 m³ zu einer Steigerung des Gasertrags von 10-15 % geführt hat. Höhere Steigerungen sind angebracht, aber konnten bisher nicht in der Praxis umgesetzt werden.

Der größte Nachteil der bekannten Verfahren ist das hohe Verstopfungsrisiko der separaten Rohrleitungen, Ultraschallreaktoren und Pumpen. Für einen sicheren Betrieb der bekannten Anwendungen sind teilweise technisch aufwendige Systeme zur Vermeidung von Verstopfungen, wie elektrisch betriebene Scherspaltzerkleinerer und sensorgestützte Überwachungssysteme, notwendig. Weiterhin gelten die hohen Investitionskosten für die Errichtung der Teilstromsysteme sowie der erforderliche Pumpenstrom als Hemmnisse für eine breite Nutzung der Ultraschalltechnologie zur Verbesserung des biologischen Abbaus in Fermentationssystemen.

Die Aufgabe der Erfindung ist eine höhere Steigerung des Gasertrags als bisher. Das angestrebte System soll kostengünstig zu realisieren sein und das Risiko einer Verstopfung vermeiden.

Diese Aufgabe wird mit einer Vorrichtung bzw. einem Verfahren nach einem der unabhängigen Patentansprüche gelöst.

Hierbei handelt es sich bei der Vorrichtung um eine Vorrichtung für den Aufschluss von biologischen Substraten unmittelbar an oder in offenen oder geschlossenen Fermentern und/oder in oder an Behältern zur Separation von Feststoffen, wobei eine oder mehrere Ultraschalleinheiten in oder an dem Fermenter und/oder in oder an dem Behälter zur Separation von Feststoffen angebracht sind und der Fermenter und/oder der Behälter zur Separation von Feststoffen für sich genommen einen Rauminhalt von mindestens 1 m³ aufweist.

Das erfindungsgemäße Verfahren wird mit einer erfindungsgemäßen Vorrichtung durchgeführt.

Bei der Vorrichtung können auch mehrere Fermenter bzw. Behälter zur Separation zur Anwendung kommen. Wichtig ist lediglich, dass mindestens einer dieser Behälter einen Rauminhalt von mehr als 1 m³, vorzugsweise mehr als 10 m³, besonders vorzugsweise mehr als 100 m³ aufweist und in eben diesem Behälter (ob geschlossen oder offen) die Ultraschalleinheiten unmittelbar unterbracht sind. Dies ist ein deutlicher Unterschied zu bestehenden Durchlauf-Systemen, bei denen beispielsweise ein rohrförmiger Abschnitt von außen beschallt wird.

Mit der erfindungsgemäßen Vorrichtung und dem erfindungsgemäßen Verfahren wird erreicht, dass eine hohe Steigerung in der Gaserzeugung erreicht werden kann, ohne dass es zu den bekannten Verstopfen und somit Betriebsausfällen kommt.

Die Anbringung der Ultraschalleinheiten innerhalb und/oder an bzw. außerhalb der Fermenter bzw. Behälter zur Separation kann unterschiedlich ausgeführt sein. So ist es möglich, dass eine Ultraschalleinheit von innen an der Fermenterwand und/oder an der Wand des Behältnisses angebracht ist und so ausgebildet ist, dass die Ultraschallschwingungen in den Fermenterraum bzw. in den Behälter zur Separation von Feststoffen hinein abgegeben werden. Es ist aber auch eine Anbringung der Ultraschalleinheiten von außen möglich.

Eine weitere Weiterbildung sieht vor, dass im Nahbereich mindestens einer Ultraschalleinheit Reflektoren positioniert sind, die ein Feld mit besonders hoher Ultraschalleistung innerhalb des Fermenters und/oder des Behälters zur Separation von Feststoffen erzeugen. Die Ultraschallleistung erreicht hierbei in einigen Bereichen Werte von mindestens 1 W/cm², vorzugsweise mindestens 5 W/cm², besonders vorzugsweise von mindestens 10 W/cm².

Eine weitere Weiterbildung sieht vor, dass die Ultraschalleinheit frei hängend oder stehend im Fermenter oder in dem Behälter zur Separation von Feststoffen positioniert ist, wobei eine schwingungsaktive Fläche der Ultraschalleinheit zur Fermenterwand und/oder zur Wand des Behälters zur Separation von Feststoffen hin angeordnet ist. Hierdurch kommt es auch zu Reflexionen an der Behälterwand bzw. zu einer relativ starken Beaufschlagung von Gut, das zwischen der Behälterwand und der Ultraschalleinheit angebracht ist.

Eine weitere Weiterbildung sieht vor, dass im Fermenter und/oder in dem Behälter zur Separation von Feststoffen mindestens ein Rührwerk zur Durchmischung des biologischen Substrats angeordnet ist. Möglich ist hierbei, dass beispielsweise ein bis fünf Rührwerke vorgesehen sind. Hierbei ist es möglich, dass diese Rührwerke auf einer gemeinsamen Welle angeordnet sind oder an verschiedenen Stellen des Fermenters bzw. des Behälters zur Separation angeordnet sind.

Eine weitere vorteilhafte Weiterbildung sieht vor, dass sich Ultraschalleinheiten auch unmittelbar auf mindestens einem der Rührwerke befinden. Hierdurch werden die Beschallung und die Durchmischung unmittelbar kombiniert und ein besonders hoher Gasertrag erzeugt.

Eine weitere Weiterbildung sieht vor, dass die Vorrichtung sowohl einen offenen oder geschlossenen Fermenter sowie vorgestaltet einen Behälter zur Separation von Feststoffen aufweist. Hierbei ist es ausreichend, wenn entweder der Fermenter oder der Behälter zur Separation Ultraschalleinheiten aufweist, vorzugsweise können allerdings auch beide Ultraschalleinheiten aufweisen.

Im Gegensatz zum Stand der Technik sind die Behälter zur Separation bzw. die Fermenter mit einem größeren Rauminhalt ausgestaltet, so sind auch Rauminhalte bis 2200 m³ und mehr möglich.

Weitere vorteilhafte Weiterbildungen werden in den übrigen Patentansprüchen angegeben.

Bei der Erfindung werden in einer Ausführungsform komplett gekapselte Ultraschall-Tauchschwinger unmittelbar im Fermenter bzw. dem Behälter zur Separation eingesetzt. Die Beschallung des Substrates erfolgt vorzugsweise mit besonders hoher Intensität im Nahbereich der angeregten Oberfläche des Schwingsystems.

In einer Beispielanordnung beträgt für die Einbringung einer vergleichbaren Energiemenge von 1kW bis 10 kW, beispielsweise 4 kW wie bei einer herkömmlichen Beschallung eines Teilstromes die schwingungsaktive Fläche des Tauchschwingers etwa 0,1 bis 1 m², beispielsweise 0,5 m². Je nach den Strömungsverhältnissen im Fermenter kann die Leistung auf mehrere im Fermenter befindliche Schwingsysteme aufgeteilt werden.

### Ausführungsbeispiele

In den folgenden Abbildungen werden verschiedene Ausführungsbeispiele gezeigt. Es zeigen:
- Abbildung 1:: An Fermenterwand montierte Tauchschwinger mit Propellerrührwerk
- Abbildung 2:: An Fermenterwand montierte Tauchschwinger mit Langachsrührwerk
- Abbildung 3:: Im Fermenter nach Außen abstrahlende Tauchschwinger
- Abbildung 4:: An Fermenterwand montierte Tauchschwinger mit Reflektor
- Abbildung 5:: Von Außen an Fermenterwand montiertes Schwingsystem
- Abbildung 6:: Offenes Gärbecken mit Tauchschwingern
- Abbildung 7:: Langachsrührwerk mit Ultraschallrührblättern
- Abbildung 8:: Aufbau Ultraschalleinheit

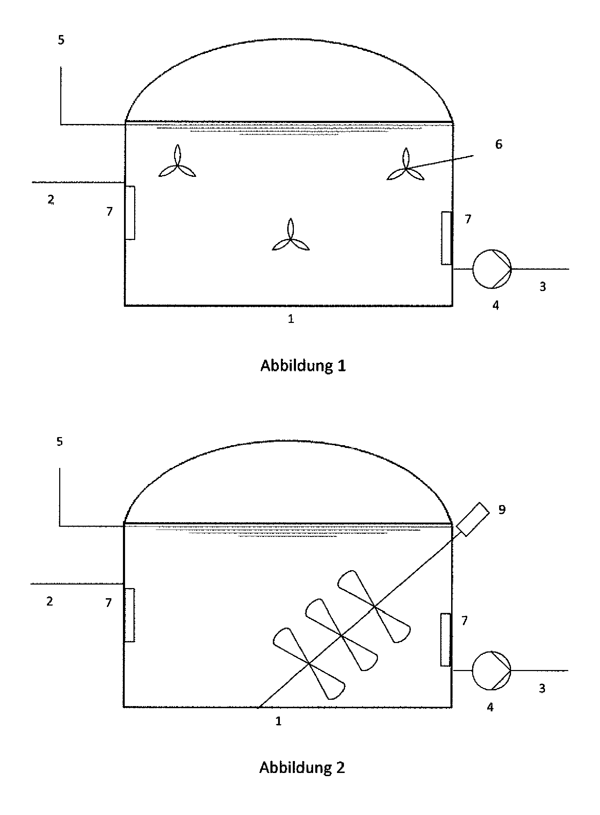

Abbildung 1 zeigt einen geschlossenen Fermenter 1 mit einem Zulauf für das Rohsubstrat 2 und einem Ablauf für das vergärte Material 3, das mit einer Pumpe 4 abgeführt wird. Über eine weitere Leitung 5 oberhalb des Substratfüllstandes wird entstehendes Biogas abgeleitet. Die Propellerrührwerke 6 ermöglichen eine Durchmischung des Fermenterinhalts und befördern den Gasaustrag aus dem Substrat. In diesem Beispiel sind die Tauchschwingersysteme 7 von Innen an die Fermenterinnenwand montiert. Die schwingungsaktive Fläche 11 zeigt dabei zur Behältermitte. Die Intensität der Ultraschallwirkung nimmt physikalisch bedingt mit steigender Entfernung zur Schwingeroberfläche ab. Insbesondere bei Substraten mit hohem Feststoffanteil aus Mais- und Grassilage ist eine gute Anströmung der Ultraschalleinheiten durch die Rührwerke entscheidend für eine umfassende Behandlung des Fermenterinhalts.

Abbildung 2 zeigt einen Fermenter mit Langachsrührwerk 9. Das Rührwerk erzeugt eine um den Behältermittelpunkt zirkulierende Kreisströmung. Die Ultraschalleinheiten 7 werden von dem Rührwerk angeströmt und ermöglichen eine kontinuierliche Beschallung des Substrates.

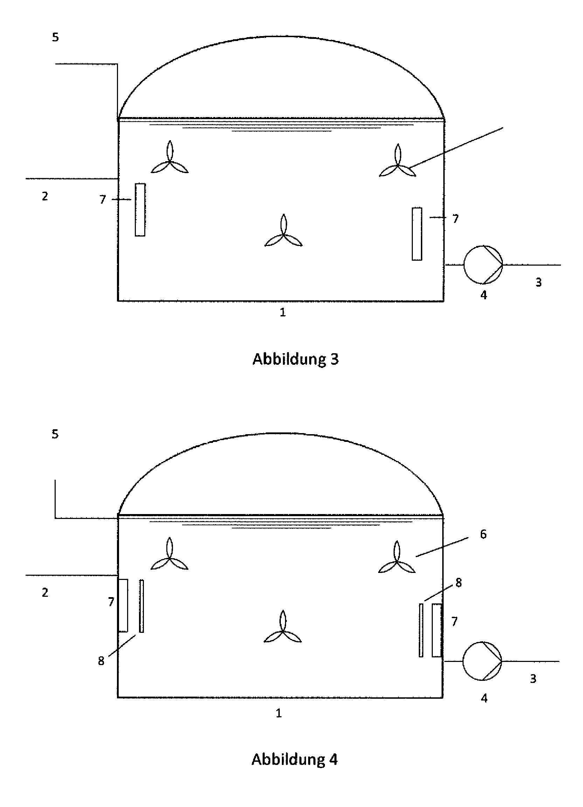

Zum Aufschluss von Substraten mit einem hohen Anteil an stabilen Verbindungen ist es erforderlich die Ultraschallleistung mit einer sehr hohen Intensität in den Fermenter einzubringen. Es ist bekannt, dass die Intensität der Kavitation bei Reflektion auf die schwingungsaktive Fläche 11 verstärkt wird. In Abbildung 3 ist die Ultraschalleinheit 7 frei hängend im Fermenter positioniert. Die schwingungsaktive Fläche 11 ist zur Behälterwand gerichtet. Die Ultraschallschwingungen werden von der Fermenterwand reflektiert und bilden ein Feld mit besonders hoher Intensität.

In Abbildung 4 sind die Ultraschallschwinger an der Fermenterwand befestigt. Die schwingungsaktive Fläche 11 zeigt zur Fermentermitte. Zur Erzeugung eines Ultraschallfeldes mit hoher Intensität sind Reflektoren 8 im Abstand von etwa 20 cm zur Ultraschalleinheit 7 positioniert.

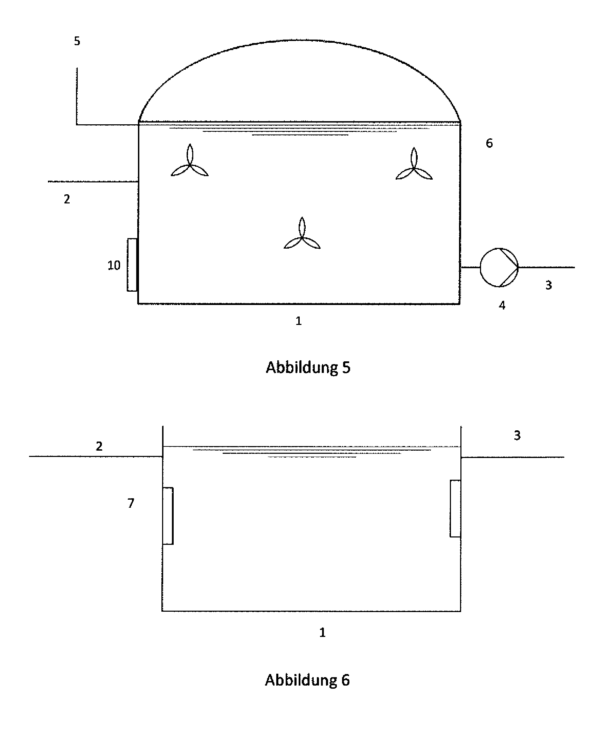

Abbildung 5 zeigt die Montage der Ultraschalleinheit 10 an der Außenseite des Fermenters. Diese Konstellation ist nur möglich, wenn die Fermenterwand aus einem Material besteht welches die Ultraschallschwingungen übertragen kann. Bei Fermentern die für eine Ultraschallübertragung ungeeignet sind, kann eine Wartungstür aus Stahl mit einer Ultraschalleinheit 10 versehen werden.

Der Vorteil von außerhalb des Fermenters angebrachten Ultraschallsystemen ist die gute Nachrüstbarkeit und die gute Zugänglichkeit bei Reparaturen.

Abbildung 6 zeigt einen offenen Fermenter 1 für die aerobe Vergärung. Diese werden beispielweise in Belebungsbecken von Kläranlage eingesetzt. Es ist bekannt, dass die Beschallung dieser Substrate mit herkömmlichen Ultraschallreaktorsystemen und anschließender Rückleitung in den Fermenter zur Verbesserung der Abbaufähigkeit geführt hat. Im Falle der beschriebenen Erfindung werden die Ultraschalleinheiten 7 an der Behälterwand montiert und geben die Ultraschallleistung in den Fermenterraum ab. Dieselbe Bauweise kann auch zu der Beschallung von Bioabfall in Separationsbecken eingesetzt werden.

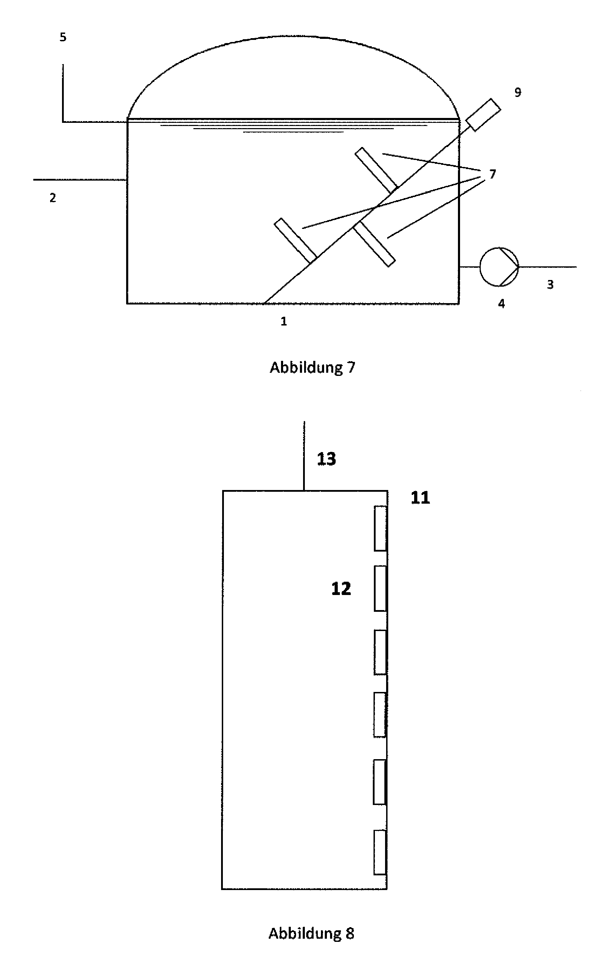

Abbildung 7 zeigt einen Fermenter 1 mit einer Kombination aus einem Langachsrührwerk 9 und mehrerer Ultraschalleinheiten 7. Die Rührblätter werden dabei durch Ultraschall angeregt und geben die Leistung direkt an das umströmende Substrat ab. Mit dieser Konstellation wird eine sehr homogene Behandlung des Fermenterinhalts ermöglicht. Durch den Ultraschall wird das umströmende Substrat zusätzlich zerkleinert und somit die notwendige Rührenergie reduziert.

Sämtliche oben beispielhaft für Fermenter genannte Ausführungsbeispiele gelten auch für beliebige offene oder geschlossene Fermenter mit einem Rauminhalt von mehr als 1 m³ sowie für Behälter im Separationsbecken.

### Bezugszeichenliste

- 1: Fermenter
- 2: Substratzuführung
- 3: Substratabführung
- 4: Substratpumpe
- 5: Gasableitung
- 6: Tauchmotorrührwerk
- 7: Ultraschalleinheit
- 8: Ultraschallreflektor
- 9: Langachsrührwerk mit Wanddurchführung
- 10: Ultraschall-Schwingplatte
- 11: Schwingungsaktive Fläche
- 12: Ultraschallschwinger
- 13: Kabelausgang

## Patentansprüche

1. Vorrichtung für den Aufschluss von biologischen Substraten unmittelbar in oder an offenen oder geschlossenen Fermentern und/oder in oder an Behältern zur Separation von Feststoffen,
wobei eine oder mehrere Ultraschalleinheiten in oder an dem Fermenter und/oder dem Behälter zur Separation von Feststoffen angebracht sind und der Fermenter und/oder der Behälter zur Separation von Feststoffen für sich genommen einen Rauminhalt von mindestens 1 m³ aufweist.

2. Vorrichtung nach Anspruch 1, **dadurch gekennzeichnet, dass** die mindestens eine Ultraschalleinheit von innen und/oder von außen an der Fermenterwand und/oder an der Wand des Behältnisses zur Separation angebracht sind und so ausgebildet sind, dass Ultraschallschwingungen in den Fermenterraum und/oder den Behälter zur Separation von Feststoffen abgegeben werden.

3. Vorrichtung nach einem der vorhergehenden Ansprüche, **dadurch gekennzeichnet, dass** im Nahbereich einer Ultraschalleinheit Reflektoren positioniert sind, die in zumindest einem Bereich ein Feld von mindestens 1 W/cm², vorzugsweise mindestens 5 W/cm², besonders vorzugsweise mindestens 10 W/cm² innerhalb des Fermenters und/oder des Behälters zur Separation von Feststoffen erzeugen.

4. Vorrichtung nach einem der vorhergehenden Ansprüche, **dadurch gekennzeichnet, dass** die Ultraschalleinheit freihängend oder stehend im Fermenter und/oder in dem Behälter zur Separation von Feststoffen positioniert ist, wobei eine schwingungsaktive Fläche der Ultraschalleinheit zur Fermenterwand und/oder zur Wand des Behälters zur Separation von Feststoffen hin angeordnet ist.

5. Vorrichtung nach einem der vorhergehenden Ansprüche, **dadurch gekennzeichnet, dass** im Fermenter und/oder in dem Behälter zur Separation von Feststoffen mindestens ein Rührwerk zur Durchmischung des biologischen Substrats angeordnet ist.

6. Vorrichtung nach Anspruch 5, **dadurch gekennzeichnet, dass** sich mindestens eine Ultraschalleinheit unmittelbar auf mindestens einem der Rührwerke befinden.

7. Vorrichtung nach einem der vorhergehenden Ansprüche, **dadurch gekennzeichnet, dass** die Vorrichtung mindestens einen offenen oder geschlossenen Fermenter sowie vorgeschaltet einen Behälter zur Separation von Feststoffen aufweist.

8. Vorrichtung nach einem der vorhergehenden Ansprüche, **dadurch gekennzeichnet, dass** der Fermenter und/oder das Behältnis zur Separation von Feststoffen einen Rauminhalt zwischen 5 m³ und 2200 m³ aufweist.

9. Verfahren für den Aufschluss von biologischen Substraten unmittelbar in offenen und/oder geschlossenen Fermentern und/oder in Behältern zur Separation von Feststoffen unter Einsatz mindestens einer Ultraschalleinheit, wobei der Rauminhalt des offenen oder geschlossenen Fermenters und/oder des Behälter zur Separation von Feststoffen mindestens 1 m³ aufweist.

10. Verfahren nach Anspruch 9, **dadurch gekennzeichnet, dass** ein Behälter zur Separation von Feststoffen sowie nachgeordnet ein offener oder geschlossener Fermenter vorgesehen sind, wobei sowohl der Behälter zur Separation von Feststoffen als auch der offene oder geschlossene Fermenter mindestens 1 m³ Rauminhalt aufweisen und in dem offenen oder geschlossenen Fermenter oder dem Behälter zur Separation von Feststoffen mindestens eine Ultraschalleinheit zum Aufschluss von biologischen Substraten vorgesehen ist.
